Europäisches Patentamt

(19) **European Patent Office**

**Office européen des brevets**

(11) Publication number: **0 067 553**
**A2**

(12) **EUROPEAN PATENT APPLICATION**

(21) Application number: **82302574.7**

(22) Date of filing: **20.05.82**

(51) Int. Cl.³: **C 12 N 15/00**
**//A01H1/00**

(30) Priority: **27.05.81 US 267539**

(43) Date of publication of application:
**22.12.82 Bulletin 82/51**

(84) Designated Contracting States:
**BE CH DE FR GB IT LI NL SE**

(71) Applicant: NATIONAL RESEARCH COUNCIL OF
CANADA

Ottawa Ontario(CA)

(72) Inventor: Pelcher, Lawrence E.
1638 Cairns Avenue
Saskatoon Saskatchewan(CA)

(72) Inventor: Halasa, Mary Christine
138 Conifer Street
Sherwood Park Alberta(CA)

(74) Representative: Pike, Harold John et al,
Abel & Imray Northumberland House 303-306 High
Holborn
London, WC1V 7LH(GB)

(54) An RNA plant virus vector or portion thereof, a method of construction thereof, and a method of producing a gene derived product therefrom.

(57) The method of construction of at least a portion of, an RNA vector from an RNA plant virus, e.g. tobacco mosaic virus (TMV) using as specific functional regions, at least two nucleotide sequences selected from oligonucleotides and polynucleotides of viral RNA for the purpose of producing an RNA molecule containing those nucleotide sequences essential for self-replication or those nucleotide sequences essential for replication upon co-infection with complete TMV in a plant cell or cells, and having the potential, in the plant cell or cells, to express and control the expression of genetic information inserted thereby and those regions necessary for the production and accumulation of stable vector particles in the plant or plant cells. The production of a gene-derived product in a plant cell from the said at least a portion of an RNA vector is also described.

EP 0 067 553 A2

Croydon Printing Company Ltd.

# AN RNA PLANT VIRUS VECTOR OR PORTION THEREOF, A METHOD OF CONSTRUCTION THEREOF, AND A METHOD OF PRODUCING A GENE DERIVED PRODUCT THEREFROM

This invention relates to an RNA plant virus vector or portion thereof, a method of construction thereof, and a method of producing a gene-derived product therefrom.

In recent years the techniques enabling the direct manipulation of DNA molecules, referred to as genetic engineering, and the discovery of bacterial DNA plasmids which, after in vitro insertion of genetic information in the form of DNA, are capable of transferring that information into bacterial and yeast cells have shown promise for the commercial production of specific gene products. Thus far, only bacterial DNA plasmids and possibly certain DNA components from yeast have proven suitable vectors or vehicles for the transfer, replication and expression of foreign genes. However, these vectors are suitable only for use in bacteria and yeast. The transfer of genetic information into higher organisms such as animal and plant cells has not been possible because of a lack of suitable vectors capable of replication in higher organisms.

Due to the rapid developments in the area of DNA engineering and gene transfer via bacterial DNA plasmids, little or no attention has been given the use of viral RNA molecules as possible vectors or vehicles for the introduction into and the replication and expression of foreign genetic information in cells.

The applicants are aware of only one publication dealing with the concept of using tobacco mosaic virus, hereinafter referred to as TMV, or components of TMV as a vector or part of a vector for the introduction of foreign genetic information into plants. This publication was entitled, "Uses of Viruses as Carriers of Added Genetic Information" by S. Rogers and P. Pfuderer, and appeared in Nature 219, 749-751, 1968, and dealt with the addition of polyadenosine, hereinafter referred to as poly A, to the 3' end of TMV-RNA and the reported subsequent expression of the poly A in inoculated tobacco plants. The authors presented data that they interpreted to indicate that the poly A added to the 3' end of the viral RNA had been expressed in inoculated tobacco plants. This

-1-

expression was in the form of polylysine (a polyamino acid or protein) reportedly synthesized as a result of infection of the plant with TMV-RNA-poly A. The results presented in this report were apparently not successfully repeated by others nor pursued by the authors of the publication.

In this specification, the 3' end is that end of the RNA molecule possessing a free hydroxyl group (OH) and the other end is the 5' end. In common usage, the 3' end refers to the right hand end of the molecule.

According to the present invention, there is provided a method of constructing an RNA plant virus vector or portion thereof from TMV and other RNA plant viruses, for the insertion of foreign genetic information therein, and the transfer into and replication and expression of the vector or portion thereof, with inserted foreign genetic information, by self-replication, or replication upon co-inoculation with helper virus, in plants or plant cells, which comprises combining a nucleotide sequence originating from the 5' end of the plus (+) strand of the viral RNA and a nucleotide sequence originating from the 3' end of the plus (+) strand of the viral RNA, such nucleotide sequences being hereinafter referred to as fragments, and being selected from the group consisting of oligonucleotides and polynucleotides:

a) the fragment originating from the 5' end (hereinafter referred to as Fragment I), and extending in the 3' end direction and comprising those nucleotide sequences complementary to the recognition and binding sites for the viral polymerase in the minus (-) strand of viral RNA, and

b) the fragment originating from the 3' end (hereinafter referred to as Fragment II), and extending in the 5' end direction and comprising those nucleotide sequences for the recognition and binding of the viral polymerase in the plus (+) strand of viral RNA, and wherein

c) at least one of the Fragments I and II contains at least a portion of the viral coat protein gene, and

-2-

d)    the Fragments I and II, alone or in combination, contain a nucleotide sequence which will control the expression of the coat protein gene,
hereinafter referred to as the control region.

In this specification, "foreign genetic information" includes
any nucleotide sequence the equivalent of which may be naturally occurring in the plant cell, to be inoculated with the vector or portion
thereof the expression of which in a plant cell, is modified by insertion
into the vector or portion thereof, or any nucleotide sequence which is
not naturally occurring in the plant cell to be inoculated with the vector or portion thereof.

Further, according to the present invention, there is provided
an RNA plant virus vector or portion thereof, derived from TMV and other
RNA plant viruses, for the insertion of foreign genetic information
therein, and the transfer into and replication and expression of the vector or portion thereof, with inserted foreign genetic information by
self-replication, or replication upon co-inoculation with helper virus,
in plants or plant cells, the RNA plant virus vector or portion thereof
comprising a nucleotide sequence originating from the 5' end of the plus
(+) strand of the viral RNA and a nucleotide sequence originating from
the 3' end of the plus (+) strand of the viral RNA, the nucleotide
sequences being hereinafter referred to as fragments, and being combined
and selected from the group consisting of oligonucleotides and polynucleotides:

a)    the fragment originating from the 5' end (hereinafter referred to as
Fragment I), and extending in the 3' end direction and comprising those
nucleotide sequences complementary to the recognition and binding sites
for the viral polymerase in the minus (-) strand of viral RNA, and

b)    the fragment originating from the 3' end (hereinafter referred to as
Fragment II), and extending in the 5' end direction and comprising those
nucleotide sequences for the recognition and binding of the viral polymerase in the plus (+) strand of viral RNA, and wherein

-3-

c)    at least one of the Fragments I and II contains at least a portion of the viral coat protein gene, and·

d)    the Fragments I and II, alone or in combination, contain a nucleotide sequence which will control the expression of the coat protein gene, hereinafter referred to as the control region.

In this specification, "an RNA plant virus vector or portion thereof derived from TMV and other RNA plant viruses" includes those constructed of nucleotide sequences originally comprising TMV and other RNA plant viruses and the progeny of RNA vectors or portions thereof, so constructed.

Further, according to the present invention, there is provided a method of producing a gene-derived product comprising, inoculating the plant or plant cell with an RNA plant virus vector or portion thereof, derived from TMV and other RNA plant viruses, the vector or portion thereof having foreign genetic information inserted therein, and being inoculated into the plant cells for the replication and expression of the vector or portion thereof, with inserted foreign genetic information, leading to the production and accumulation of the said gene-derived product, by self-replication, or replication upon co-inoculation with helper virus, in plants or plant cells, the RNA plant virus vector or portion thereof comprising a nucleotide sequence originating from the 5' end of the plus (+) strand of the viral RNA and a nucleotide sequence originating from the 3' end of the plus (+) strand of the viral RNA, the nucleotide sequences being hereinafter referred to as fragments, and being combined and selected from the group consisting of oligonucleotides and polynucleotides:

a)    the fragment originating from the 5' end (hereinafter referred to as Fragment I), and extending in the 3' end direction and comprising those nucleotide sequences complementary to the recognition and binding sites for the viral polymerase in the minus (-) strand of viral RNA, and

b)    the fragment originating from the 3' end (hereinafter referred to as Fragment II), and extending in the 5' end direction and comprising those

-4-

nucleotide sequences for the recognition and binding of the viral polymerase in the plus (+) strand of viral RNA, and wherein

c) at least one of the Fragments I and II contains at least a portion of the viral coat protein gene, and

d) the Fragments I and II, alone or in combination, contain a nucleotide sequence which will control the expression of the coat protein gene, hereinafter referred to as the control region.

In some embodiments of the present invention the Fragments I and II, alone or in combination, contain a nucleotide sequence or portion thereof necessary for the encapsidation of the RNA by viral coat protein, the sequence being hereinafter referred to as the nucleation region.

In some embodiments of the present invention, there is an insertion or attachment of foreign genetic information, ultimately in the form of RNA, to the said RNA vector or portion thereof.

In some embodiments of the present invention, the foreign genetic information is inserted into or attached to the coat protein gene or portion thereof.

In some embodiments of the present invention, the foreign genetic information is inserted into or attached to the control region or portion thereof.

In some embodiments of the present invention, the foreign genetic information in inserted into or attached to the nucleation region or portion thereof.

In some embodiments of the present invention, the RNA plant virus vector or portion thereof is for the purpose of reproduction by inoculation into plants or plant cells.

In some embodiments of the present invention, the said RNA plant virus vector or portion thereof with added foreign genetic information is for the purpose of reproduction by inoculation into plants or plant cells.

In some embodiments of the present invention, the said RNA plant virus vector or portion thereof, with added foreign genetic information, is for the purpose of directing the synthesis of a product, selected from the group consisting of proteins, oligonucleotides, polynucleotides, peptides, enzymes, antibodies, antigenic substances, antiviral compounds, anti-cancer compounds, and primary and secondary metabolites upon inoculation into plants or plant cells.

In some embodiments of the present invention, the said RNA plant virus vector or portion thereof, with added foreign genetic information, is for the purpose of altering the metabolic or catabolic capability of plants or plant cells upon inoculation into plants or plant cells.

In some embodiments of the present invention, the said RNA plant virus vector or portion thereof, with added foreign genetic information, is for the purpose of altering at least one of the group consisting of growth habit, yield potential, disease resistance, resistance to environmental stress and energy utilization of the plants or plant cells, upon inoculation into plants or plant cells.

Some embodiments of the present invention are concerned with the construction of at least a portion of an RNA plant virus vector from TMV which will enable the transfer into, and the replication and expression of foreign genetic information in plants or plant cells. Novel features of the said at least a portion of a plant virus vector are believed to be:

1) The said at least a portion of an RNA plant virus vector is constructed from tobacco mosaic virus (TMV) and other RNA plant viruses.

2) This said at least a portion of a plant virus vector will enable the transfer of genetic information into and the replication and expression of this information in plants or plant cells.

3) The inserted genetic information will be in the form of RNA rather than DNA, as in bacterial plasmids.

4) The said at least a portion of a plant virus vector wtih inserted

-6-

genetic information in the form of RNA is in some embodiments of the present invention, constructed in such a manner as to utilize a unique capability of TMV, namely to direct the synthesis of extremely large amounts of a single protein.

In the accompanying drawings which illustrate, by way of example, embodiments of the present invention:

Figures 1 and 2 together depict a flow diagram of the basic steps involved in the replication of complete TMV and related strains of virus,

Figure 3 depicts diagrams showing how replication of TMV, and related strains of virus, is thought to proceed and the possible effects that the addition of genetic information to the 3' end may have on replication,

Figure 4 is a diagrammatic view of nucleotide sequences (Fragments I and II) from TMV, and related strains of viruses, which may be used for the construction of a plant virus vector, or portion thereof,

Figure 5 is a flow diagram of the construction of an at least a portion of an RNA plant virus vector from TMV and related strains of virus by the deletion of at least a portion of the coat protein gene and replacement of said gene or a portion thereof by RNA nucleotide sequences coding for the synthesis of a desired product,

Figure 6 is a flow diagram of the construction of an at least a portion of an RNA plant virus vector from TMV and related strains of virus having at least a portion of the nucleation region deleted to prevent the encapsidation of the RNA vector or portion thereof by viral coat protein,

Figures 7 to 9 together depict a flow diagram for the generation and isolation of specific nucleotide sequences (Fragments I and II) from TMV, and the construction of an RNA vector or portion thereof therefrom, through to the recovery of the replicated vector or portion thereof from

-7-

infected plants or plant cells, these figures also depict the insertion of foreign genetic information into the RNA vector or portion thereof and the subsequent expression of the foreign genetic information resulting in the synthesis of a gene-derived product in plants or plant cells,

Figures 10 and 11 together depict a different flow diagram for the generation and isolation of specific nucleotide sequences (Fragments I and II) from TMV, and the construction of an RNA vector or portion thereof therefrom, through to the recovery of the replicated vector or portion thereof from infected plants or plant cells, these figures also depict the insertion of foreign genetic information into the RNA vector or portion thereof and the subsequent expression of the foreign genetic information resulting in the synthesis of a gene-derived product in plants or plant cells, and

Figures 12 to 15 together depict a different flow diagram for the generation and isolation of specific nucleotide sequences (Fragments I and II) from TMV, and the construction of an RNA vector or portion thereof therefrom, through to the recovery of the replicated vector or portion thereof from infected plants or plant cells, these figures also depict the insertion of foreign genetic information into the RNA vector or portion thereof and the subsequent expression of the foreign genetic information resulting in the synthesis of a gene-derived product in plants or plant cells.

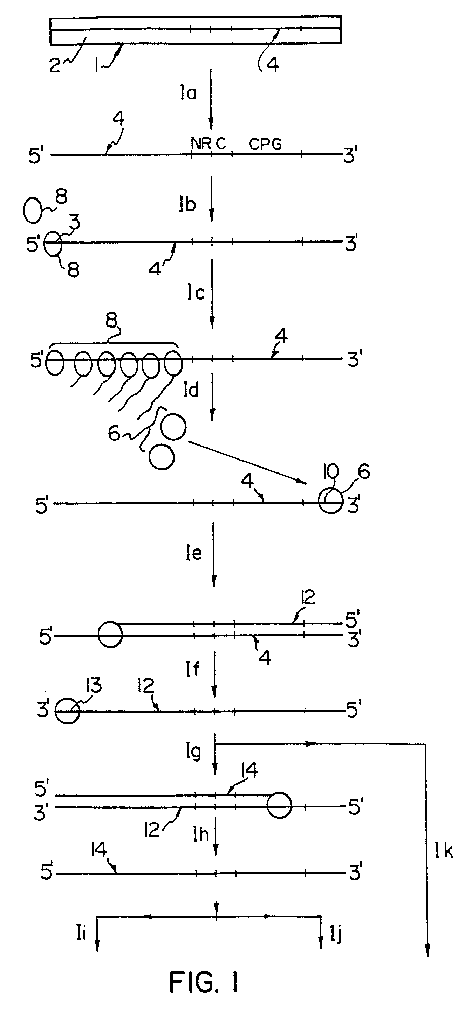

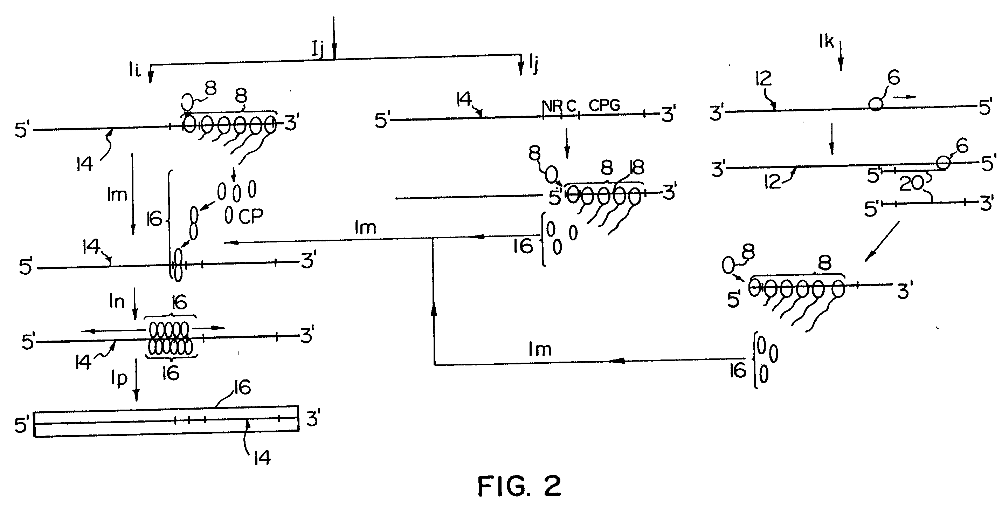

In figures 1 and 2, NR is the nucleation region, C is the control region for the expression of the coat protein gene, and CPG is the coat protein gene.

Referring now to figures 1 and 2, TMV, and related strains in the form of virus particles, generally designated 1, enters plant cells (not shown) through natural or artificial openings. During or shortly after entry into the cells, the viral coat protein 2 is removed (disassembly)(1a), freeing the viral RNA 4. The viral RNA 4 from the infecting virus particle is hereinafter referred to as the plus (+) strand. The (+) strand 4 initially functions as messenger RNA (m-RNA) for the

-8-

synthesis of the viral replicase enzyme(s) 6 or for a viral protein(s) which modify existing or induced cellular RNA polymerases in such a manner as to function as viral replicase(s) (1b, 1c and 1d). As will be described later, this initial functioning of the (+) strand 4 as m-RNA requires a leader sequence in the form of a ribosome 8 binding site 3 and may require a cap structure ($m^7$GpppG) at the 5' end.

Following synthesis of the viral replicase 6, the (+) strand 4 functions as a template for the synthesis of the minus (-) strands, such as 12, which is complementary in nucleotide sequence to the (+) strand 4. Binding of the viral replicase or polymerase 6 to the (+) strand is effected by recognition of a nucleotide sequence 10 at or near the 3' end of viral RNA 4 (1e). After or during completion of the synthesis of the (-) strands, such as 12, (1f), the viral polymerase 6 recognizes a sequence 13 at or near the 3' end of the (-) strands, such as 12, (1g), which is complementary to the 5' end of the (+) strand 4. This recognition results in the (-) strands, such as 12, functioning as a template for the synthesis of (+) strands, such as 14, of viral RNA (1h).

The synthesis of viral coat protein 16 is accomplished by using (+) strands, such as 14, either directly as m-RNA (1i) or portions thereof in the form of fragments 18 generated by processing (1j). The synthesis of viral coat protein 16 may also be accomplished by using (-) strands such as 12, to direct the synthesis of less than full-length portions 20 of a (+) strand, which function as m-RNA for the synthesis of coat protein 16 (1k). Translation of the coat protein gene (CPG), in whatever form the m-RNA takes, requires the recognition of a specific nucleotide sequence by cellular ribosomes 8. This sequence likely lies immediately prior to [i.e. to the left of in figures (1i), (1j) and (1k)] the sequences which direct the synthesis of the viral coat protein. The coat protein is produced in far greater amounts than other viral gene products. Based on recovery of complete virus particles from infected plants, the yield of coat protein ranges from 1-10 g/kg fresh weight of leaf material (complete TMV is 95% protein and 5% RNA).

-9-

In some varieties of tobacco, the virus is able to spread and replicate throughout the plant (systemic infection) after entry into one or more cells at the site of infection. It is this systemic invasion which in large part accounts for the high yield of virus obtainable.

Subsequent to coat protein synthesis, these proteins recognize and bind to a specific nucleotide sequence at approximately 1000 nucleotides from the 3' end of viral RNA molecules. This sequence is referred to as the nucleation region (NR). Binding then proceeds in both directions from this region. Thus the viral RNA is encapsidated (coated with viral proteins) and complete virus accumulates in the cytoplasm of the infected cell.

The RNA plant virus vector or portion thereof, from TMV and related strains of virus will follow the same basic steps that are involved in the synthesis of complete TMV and related strains of virus as those described with reference to figures 1 and 2.

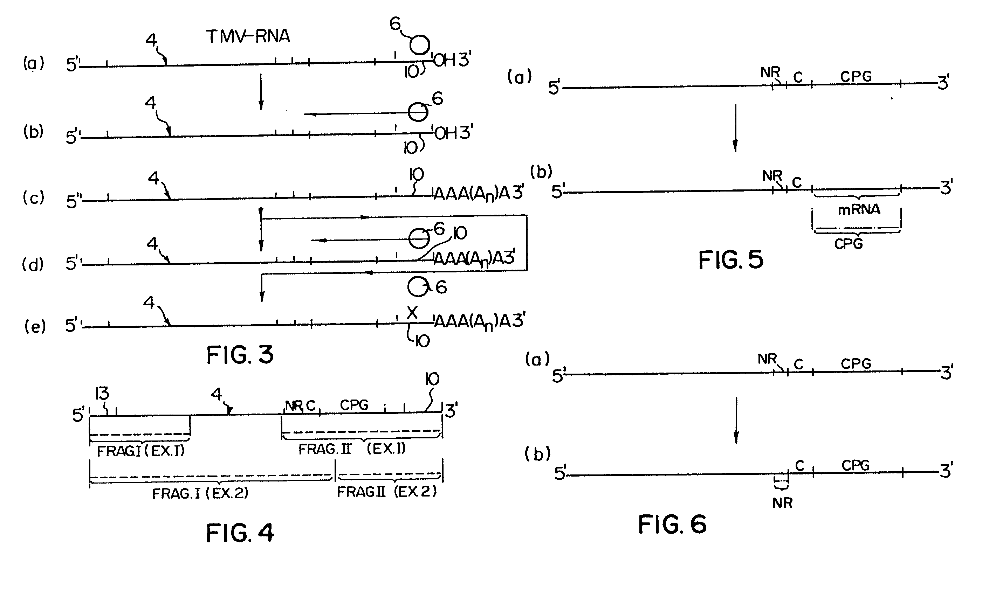

In figure 3, similar parts to those shown in figures 1 and 2 are designated in the same manner and the previous description is relied upon to describe them.

Based on the generally accepted understanding of the events involved with RNA plant virus replication in general, and TMV replication in particular, at the time of filing this patent application, while the contributions by Rogers and Pfuderer were useful, their teachings have not led to an accepted technique for the insertion or addition of foreign genetic information, in the form of RNA, into tobacco mosaic virus and other RNA plant viruses, or the subsequent use of this virus as vectors for the introduction into and replication and expression of foreign genetic information in plants or plant cells, for the following reason.

Referring to figure 3, addition of genetic information, in the form of poly A, to the 3' end of the viral RNA 4 is likely not to have been replicated by the viral replicase enzyme 6. This enzyme presumably recognizes a specific site (unique sequence of nucleotides) 10 at or near the 3' end of the viral RNA molecule (3a). Replication is then believed to proceed from the 3' end of the viral RNA toward the 5' end (left hand end) of the viral RNA (3b). Because the poly A claimed added by Rogers and Pfuderer would have been to the right hand side of the replicase binding site 10 and to the right of the 3' end of the viral RNA (3c), the poly A sequence would not have been replicated and thus would have been lost for subsequent steps in virus synthesis (3d). The loss of the poly A at this stage would have precluded continued expression of the added information within the plant. This is supported by the observation that poly A added to the 3' end of bacteriophage Qβ RNA is not replicated in or expressed by infected bacteria and, therefore, subsequently not present in progeny viral RNA. (See C. Gilvarg et al, Proc. Nat. Acad. Sci. U.S.A., 72, 428–432, 1975.) An alternative to this is that TMV-RNA molecules, modified by 3' end poly A addition, may not be replicated in the plant (3e). This is supported by the observation that bacteriophage Qβ RNA, modified by poly A addition to the 3' end of the molecule, is not replicated in vitro by purified viral replicase (see R. Devos et al, Biochem. et Biophys. Acta 447, 319–327, 1976).

Based on the above analysis and supporting literature, it can be concluded that the approach of Rogers and Pfuderer could not be applied to the use of plant RNA viruses as vectors or vehicles for the introduction of foreign genetic information into plants. Further, as will be seen from the following, the Rogers and Pfuderer disclosure would lead a person skilled in the art away from the introduction of specific genetic information into TMV vectors according to the present invention.

As previously stated, the Rogers and Pfuderer disclosure is the only one of which the applicants are aware which may be interpreted to relate to the specific use of RNA plant viruses (such as TMV and related strains) as vectors for the introduction, replication and expression of foreign genetic information into plants or plant cells.

In figure 4, similar parts to those shown in figures 1 and 2 are designated in the same manner and the previous description is relied

-11-

upon to describe them.

Referring now to figure 4, certain aspects of the present invention are concerned with the construction from specific nucleotide sequences (Fragments I and II) of viral RNA derived from tobacco mosaic virus (TMV) or related strains to form a vector or portion thereof for the introduction of foreign genetic information into, and the subsequent replication and expression of this information in plants or plant cells. The vector or portion thereof may be constructed from the following viral RNA nucleotide sequences and techniques:

1) Fragment I. A nucleotide sequence or fragment which originates from the 5' end of the viral RNA, the actual size (nucleotide length) of this fragment to depend on the specific approach used to generate this fragment. This nucleotide sequence is designated Frag. I (Ex.1) or Frag. I (Ex. 2) in figure 4, each of which is shown dashed when generated.

2) Fragment II. A nucleotide sequence or fragment which originates from the 3' end of the viral RNA, the actual size (nucleotide length) of this fragment to depend on the specific approach used to generate this fragment. This nucleotide sequence is designated Frag. II (Ex.1) or Frag. II (Ex. 2) in figure 4, each of which is shown dashed when generated.

3) The cleavage of nucleotide sequences present in a vector or portion thereof, at specific sites in such a manner as to allow the introduction of foreign genetic information into those sites.

4) The insertion and method of insertion of foreign genetic information into or immediately adjacent to the coat protein gene, as will be described later.

Requirements for Construction of a Functional RNA Plant Virus Vector or a Portion thereof, from RNA Plant Viruses such as TMV and Related Strains of Virus:

For the RNA vector or portion thereof to be functional, it must contain those nucleotide sequences essential for replication (recognition

-12-

by viral polymerase), expression and control of expression of inserted genetic information, examples of which are:

a)   the fragment originating from the 5' end (hereinafter referred to as Fragment I), and extending in the 3' end direction and comprising those nucleotide sequences complementary to the recognition and binding sites for the viral polymerase in the minus (-) strand of viral RNA, and

b)   the fragment originating from the 3' end (hereinafter referred to as Fragment II), and extending in the 5' end direction and comprising those nucleotide sequences for the recognition and binding of the viral polymerase in the plus (+) strand of viral RNA, and wherein

c)   at least one of the Fragments I and II contains at least a portion of the viral coat protein gene, and

d)   the Fragments I and II, alone or in combination, contain a nucleotide sequence which will control the expression of the coat protein gene, hereinafter referred to as the control region.

It should be noted that the above mentioned "essential" nucleotide sequences may be present in their original or a modified form.

In figures 5 and 6, similar parts to those shown in figures 1 to 4 are designated in the same manner and the previous description is relied upon to describe them.

Functional Considerations Which May be Required to Meet a Special Need:

•The nucleotide sequences of the viral RNA described above may, in some embodiments of the present invention, be present in the RNA plant virus vector or portion thereof, and are therefore considered an integral part thereof. However, in some embodiments of the present invention, some of these nucleotide sequences may be modified or deleted in part or in whole to meet a particular functional application of the RNA plant virus vector;

a)   To meet the functional requirements for the synthesis of a free-formed product (not covalently attached to a portion of the coat protein

-13-

molecule), it may be necessary to delete a part or all of those nucleotide sequences (CPG) which code for the synthesis of coat protein. However, as shown in figure 5, that nucleotide sequence, designated C,
necessary for the control of coat protein synthesis is retained in the
RNA plant virus vector while the coat protein gene nucleotide sequence,
designated CPG and shown chain dotted thus —··—, is deleted in part or in
whole and is replaced by an RNA nucleotide sequence(s) (e.g. mRNA as
shown in figure 5) for directing synthesis of the desired product.

b) To meet the functional requirements of biological containment due to
the nature of the inserted genetic information, it may be necessary to
prevent the formation of stable vector particles. As shown in figure 6,
to meet this requirement, the nucleation region, designated NR and shown
chain dotted thus —···—, may be deleted in part or whole from the RNA
plant virus vector or portion thereof.

Thus it may be desirable or necessary to delete in part or
whole or to otherwise modify certain nucleotide sequences of the RNA
plant virus vector or portion thereof to meet specific functional
requirements. Such deletions or modifications, however, would not change
the nature of the RNA plant virus vector from that which would be generally referred to in the art as an RNA plant virus vector or portion
thereof.

### Examples of the Present Invention

#### Example I

##### A. Generation and Isolation of Nucleotide Sequence Fragments of TMV-RNA
Direct Construction of the RNA Vector by RNA-RNA Ligation - Reverse-
Transcription of the RNA Vector - Insertion of the DNA Copy into Bacterial Plasmids - Insertion of a DNA Copy of Foreign Gene into the Vector
Coat Protein Gene - Recovery of the RNA Vector Containing RNA Copy of the
Inserted Gene (hereinafter referred to as V-RNA) - Replication of the RNA
Vector (V-RNA) and Expression of the Inserted Gene in Plants, for example

-14-

Tobacco or Tomato Plants - Recovery of the V-RNA Particles from Infected
Plants.

Fragment I Generation and Isolation:  TMV-RNA is isolated from TMV using
known phenol extraction techniques.  The RNA is then specifically methylated at the second G (guanosine) residue of the cap structure at
the 5' end of the RNA (m7GpppGU... ———→m7GpppGmU...).  Methyl transferase
purified from vaccinia virions is used to accomplish this methylation.
The methylated RNA is then extensively digested with ribonuclease $T_1$
(Cal Biochem, La Jolla, California, U.S.A.).  The large RNA fragment
originating from the 5' end of the viral RNA is then isolated using
standard procedures.  This fragment, hereinafter referred to as Fragment
I, is approximately 71 nucleotides in length and terminates at the 5' end
with an intact cap structure.  The 3' terminal phosphate of this fragment
removed by alkaline phosphatase (P-L Biochemicals Inc., Milwaukee,
Wisconsin, U.S.A.) treatment.  The nucleotide sequence of Fragment I may
be as follows:

## Fragment I

```
|-Cap-|---------------Leader Sequence----------
|     |      10      20      30      40
m7GpppGUAUUUUUACAACAAUUACCAACAACAACAAACAACAAACAA


-------------------------------------|
     50      60      70|
CAUUACAAUUACUAUUUACAAUUACAAUG   3'
```

Fragment II Generation and Isolation:  Complete TMV (1 volume) is treated
with 200 mM 2-amino-2-methyl-1,2-propanediol, 20 mM NaCl, pH 9.5 (1 volume) for 18 hours at about 0°C.  Magnesium chloride (200 mM) is then
added to a final concentration of 1 mM.  Neurospora crassa nuclease (P-L
Biochemicals Inc.) is then added (5 U/ml) and incubation at about 0°C
continues for a further 18-24 hr period.  The intact ribonucleoprotein
particles, containing the RNA, hereinafter referred to as Fragment II,
are then isolated and purified.  Fragment II is then isolated by phenol

-15-

extraction techniques. Fragment II, produced as described above, has a 5' terminal phosphate. The nucleotide sequence of Fragment II may be as follows on page 17, [Note: the underlined nucleotide sequences are the nucleotide sequences corresponding to the Sst I restriction sites in the double-stranded DNA copy in Example I as will be described later].

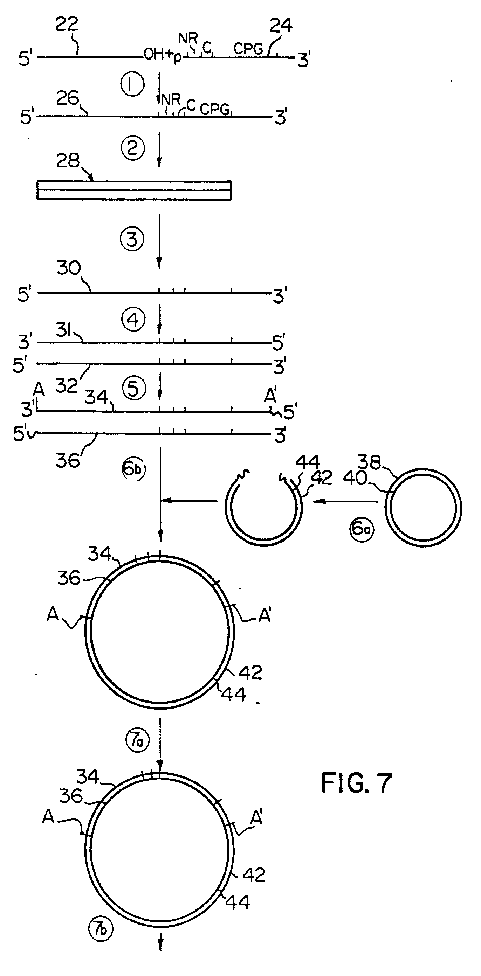

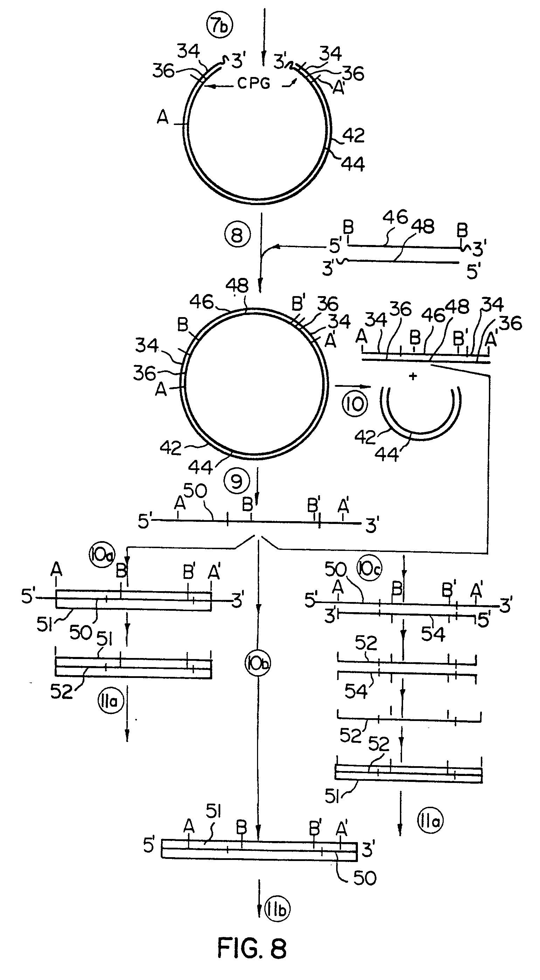

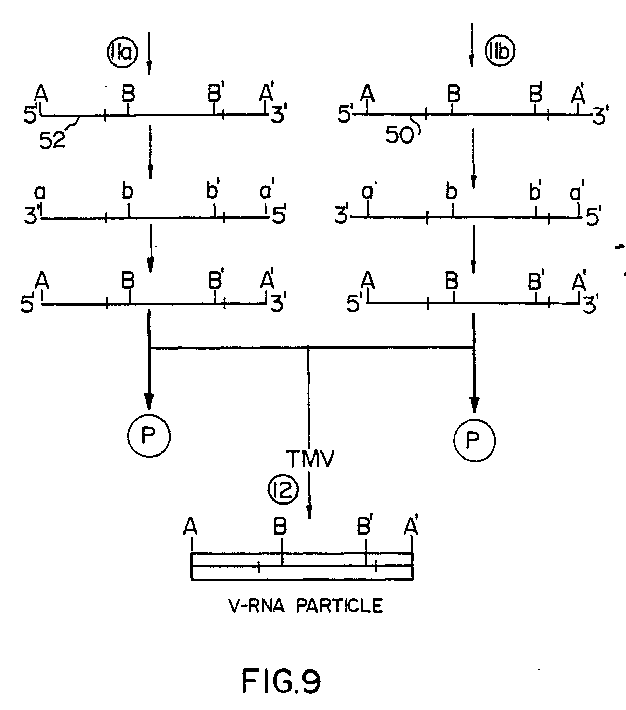

In figures 7 to 9, similar parts to those shown in figures 1 to 5 are designated in the same manner and the previous description is relied upon to describe them.

Direct Construction of an RNA Plant Virus Vector From TMV By RNA-RNA Ligation:

In figures 7 to 9, Fragments I and II, designated 22 and 24 respectively, are mixed together in an appropriate buffer and incubated with RNA ligase (100-200 U/ml) (P-L Biochemicals Inc., Milwaukee, Wisconsin, U.S.A.) at 4-10°C for 1-3 days in step 1, designated ①. Ligation of these Fragments I and II results in the formation of the RNA molecule referred to as an RNA vector or portion thereof (V-RNA) and designated in figure 7 as 26. The efficiency of the ligation reaction may be extremely low. Recovery of the V-RNA is accomplished by incubating the reaction mixture with TMV coat protein in known manner. RNA molecules containing the nucleation region will be encapsidated by the protein. The V-RNA particles (encapsidated V-RNA) can then be purified in step 2, designated ②, using standard procedures to provide vector particles or portions thereof (V-particles) designated 28. The vector particles are then, in step 3 (designated ③), co-inoculated into tobacco along with complete TMV (the complete virus directs synthesis of viral replicase needed for V-RNA replication), and after replication (amplification) the vector particles are extracted and purified by standard procedures and the V-RNA designated 30 is then isolated by phenol extraction techniques.

-16-

Fragment II

5' AUUGUUUAUAGAAAUAAUAUAAAAAUUAGGUUUGAGAGAGAAGAUUACAAGCGUGAGAGACGGAGGG

CCCAUGGAACUUACAGAAGAAGUUGUUGAUGAGUUCAUGGAAGAUGUCCCUAUGUCAAUCAGACUU

—————Nucleation Region—————

GCAAAGUUUCGAUCUCGAACCGGAAAAAAGAGUGAUGUCCGUAAAGGGAAAAUUAGUAGUAGUGAU

————Control Region————————

CGGUCAGUGCCGAACAAGAACUAUAGAAAUGUUAAGGAUUUUGGAGGAAUGAGUUUUAAAAAGAAU

AAUUUAAUCGAUGAUGAUUCGGAGGCUACUGUCGCCGAAUCGGAUUCGUUUUAAAUAUGUCUUACA

GUAUCACUACUCCAUCUCAGUUCGUGUUCUUGUCAUCAGCGUGGGCCGACCCAAUAGAGUUAAUUA

AUUUAUGUACUAAUGCCUUAGGAAAUCAGUUUCAAACACAACAAGCUCGAACUGUCGUUCAAAGAC

AAUUCAGUGAGGUGUGGAAACCUUCACCACAAGUAACUGUUAGGUUCCCUGACAGUGACUUUAAGG

—————Coat Protein Gene—————

UGUACAGGUACAAUGCGGUGUUAGACCCGCUAGUCACAGCAUUACUAGGUGCAUUUGACACUA

GAAAUAGAAUAAUAGAAGUUGAAAAUCAGGCGAACCCCACGACUGCCGAAACGUUAGAUGCUACUC

GUAGAGUAGACGACGCGACGGUGGCCAUAAGGAGCGCGAUAAAUAAUUUAAUAGUAGAAUUGAUCA

GAGGAACCGGAUCUUAUAAUCGGAGCUCUUUUCGAGAGCUCUUCUGGUUUGGUUUGGACUUCCGGUC

CUGCAACUUGAGGUAGUGCAACUUGAGGUAGUCAAGAUGCAUAAUAAAUAACGGAUUGUGUCCGUA

AUCACACGUGGUGCGUACGAUAACGCAUAGUGUUUUUUCCCUCCACUUAAAUCGAAGGGUUGUGUCU

UGGAUCGCGCGGGUCAAAUGUAUAUGGUUCAUAUACAUCCGCAGGCACGUAAUAAAGCGAGGGGUU

CGAAUCCCCCCGUUACCCCCGGUAGGGGCCCA—OH     3'

—17—

## Reverse Transcription Of The RNA Vector:

The V-RNA 30, appropriately primed at its 3' end with a short complementary oligodeoxynucleotide, is reverse-transcribed in step 4, designated ④, with avian myeloblastosis virus RNA-dependent DNA polymerase (reverse transcriptase) [Bethesda Research Laboratories Inc., Bethesda, Maryland, U.S.A., (BRL)], thus forming a single-stranded DNA copy 31 of the V-RNA, hereinafter referred to as c-V-DNA. Also, in step 4, designated ④, the c-V-DNA is isolated after alkaline treatment which degrades the V-RNA, the c-v-DNA is then copied using DNA polymerase I (P-L Biochemicals Inc., Milwaukee, Wisconsin, U.S.A.) thus forming a double-stranded DNA copy 31,32 of the V-RNA, hereinafter referred to as dc-V-DNA. In step 5, designated ⑤, the dc-V-DNA is then appropriately modified in known manner at its ends to form dc-V-DNA 34,36 with ligatable linker sequences.

## Insertion Of The DNA Copy Into Bacterial Plasmids:

Purified bacterial plasmids (e.g. pBR322 -BRL) in the form of closed circular double-stranded DNA designated 38,40 are treated in step 6a, designated ⑥ₐ, with the appropriate restriction endonuclease to cleave the plasmid at a single site thus opening the circular plasmid structure to form the structure designated 42,44, with ends complementary to those of the dc-V-DNA 34,36. For example, restriction endonuclease EcoRI cleaves pBR322 at a single site with the nucleotide sequence

$$5' \overset{\downarrow}{-\text{GAATTC}}$$

$$3' -\text{CTTAA}\underset{\uparrow}{\text{G}}$$

In step 6b, designated ⑥ᵦ, after cleavage, the open circular plasmid structure 42,44 and dc-V-DNA 34,36 are incubated together in the presence of DNA ligase (BRL). In the presence of this enzyme, the dc-V-DNA 34,36 is covalently inserted into the cleaved portion of the plasmid. In step 7a, designated ⑦ₐ, the dc-V-DNA plasmid 34, 36, 42, 44, is then replicated, selected, and amplified in a suitable strain of bacteria, such as, for example, Escherichia coli (E. coli).

-18-

Insertion Of A DNA Copy Of The Foreign Gene Into The Vector Coat Protein Gene:

The purified dc-V-DNA plasmid 34, 36, 42, 44 is treated in step 7b, designated (7b), with an appropriate restriction endonuclease to specifically cleave the dc-V-DNA plasmids 34, 36, 42, 44, in that region of the dc-V-DNA corresponding to the TMV coat protein gene, designated CPG. For example, restriction endonuclease Sst I (BRL) will cleave at the nucleotide sequence
$$5' \text{-GAGC}\overset{+}{\text{T}}\text{C}$$
$$3' \text{-}\underset{+}{\text{C}}\text{TCGAG}$$
This sequence occurs twice in the dc-V-DNA plasmid 34, 36, 42, 44, both sites lying within the TMV coat protein gene. The sites of cleavage are in the nucleotide sequences which code for amino acids 141-143 and 145-147 in the TMV coat protein molecule (see Fragment II above for the corresponding sites in the coat protein gene and the following amino acid sequence for the corresponding sites in the TMV coat protein molecule).

### Amino Acid Sequence of TMV Coat Protein

```
        1     2    3    4    5    6    7    8    9    10   11   12   13   14   15   16
Acetyl-Ser-Tyr-Ser-Ile-Thr-Thr-Pro-Ser-Gln-Phe-Val-Phe-Leu-Ser-Ser-Ala-

17  18   19   20   21   22   23   24   25   26   27   28   29   30   31   32   33   34
Try-Ala-Asp-Pro-Ile-Glu-Leu-Ile-Asn-Leu-Cys-Thr-Asn-Ala-Leu-Gly-Asn-Gln-

35  36   37   38   39   40   41   42   43   44   45   46   47   48   49   50   51   51
Phe-Gln-Thr-Gln-Gln-Ala-Arg-Thr-Val-Val-Gln-Arg-Gln-Phe-Ser-Gln-Val-Try-

53  54   55   56   57   58   59   60   61   62   63   64   65   66   67   68   69   70
Lys-Pro-Ser-Pro-Gln-Val-Thr-Val-Arg-Phe-Pro-Asp-Ser-Asp-Phe-Lys-Val-Tyr-

71  72   73   74  .75  76   77   78   79   80   81   82   83   84   85   86   87   88
Arg-Tyr-Asn-Ala-Val-Leu-Asp-Pro-Leu-Val-Thr-Ala-Leu-Leu-Gly-Ala-Phe-Asp-

89  90   91   92   93   94   95   96   97   98   99  100  101  102  103  104  105  106
Thr-Arg-Asn-Arg-Ile-Ile-Glu-Val-Glu-Asn-Gln-Ala-Asn-Pro-Thr-Thr-Ala-Glu-

107 108  109  110  111  112  113  114  115  116  117  118  119  120  121  122  123  124
Thr-Leu-Asp-Ala-Thr-Arg-Arg-Val-Asp-Asp-Ala-Thr-Val-Ala-Ile-Arg-Ser-Ala-

125 126  127  128  129  130  131  132  133  134  135  136  137  138  139  140  141  142
Ile-Asn-Asn-Leu-Ile-Val-Glu-Leu-Ile-Arg-Gly-Thr-Gly-Ser-Tyr-Asn-Arg-Ser-

143 144  145  146  147  148  149  150  151  152  153  154  155  156  157  158
Ser-Phe-Glu-Ser-Ser-Ser-Gly-Leu-Val-Try-Thr-Ser-Gly-Pro-Ala-Thr
```

_____ sequence corresponding to Sst I restriction sites in dc-V-DNA in Ex. I.

_ _ _ _ sequence corresponding to that cleaved by ribonuclease H in Ex. II.

-19-

After cleaving the coat protein gene of dc-V-DNA, a dc-DNA copy of a gene 46, 48 with gene limits B-B', which has been appropriately modified at its 3' ends, is covalently inserted in step 8, designated ⑧, into the coat protein gene of the dc-V-DNA using DNA ligase. The plasmid so constructed is allowed in step 9, designated ⑨, to replicate in the appropriate bacteria and at the appropriate time total RNA is extracted from the plasmid-infected bacteria.

Recovery Of The V-RNA Containing An RNA Copy Of The Inserted Gene:

During replication of the plasmid in the bacteria, plasmid-directed RNA synthesis occurs. Some of the RNA synthesized in step 9 will include copies of the dc-V-DNA containing the inserted gene. This RNA, designated 50, combines V-RNA (A-A') and covalently attached plasmid RNA (5'-A and A'-3'). This RNA 50 can be specifically isolated by incubation of the total RNA with TMV coat protein, designated 51. The coat protein 51 will bind to and encapsidate only the RNA 50, containing the nucleation region present in V-RNA. However, as shown in 10a, the encapsidation may not extend beyond the 3' and 5' ends of the V-RNA, thus plasmid RNA beyond these points will not be encapsidated. In this case, the exposed plasmid RNA is removed by nuclease treatment thus producing a particle 51, 52 composed only of V-RNA and TMV coat protein.

On the other hand, the encapsidation may extend beyond the 3' and 5' ends of the V-RNA to include the covalently attached plasmid RNA (as shown in 10b). In this case, the V-RNA would be recovered when the particle is replicated, as will be described later.

A second approach for specific isolation of V-RNA from the total RNA extracted from plasmid-infected bacteria is to mix in step 10c, the RNA with the dc-V-DNA recovered from purified dc-V-DNA plasmids by, for example, Eco RI treatment. This mixture is then heat-denatured and allowed to cool slowly; during the cooling period, single-stranded c-V-DNA 54 complementary to V-RNA will anneal with the V-RNA A-A' portion of RNA 50, 54, thus forming a base-paired hybrid c-V-DNA/V-RNA. Plasmid

-20-

RNA, possibly attached to the 3' and/or 5' end of the V-RNA, will remain single-stranded. This single-stranded RNA can be degraded using single-strand specific nuclease (e.g. $S_1$ Nuclease, P-L Biochemicals Inc.). After purification, the DNA strand 54 of the c-V-DNA/V-RNA hybrid can be degraded with DNAase. The intact V-RNA 52 can then be encapsidated with TMV coat protein 51. The V-RNA particle 51, 52 corresponds to those recovered by the procedures outlined in 10a above.

Replication Of The RNA Vector And Expression Of The Inserted Gene In Plants or Plant Cells, For Example, Tobacco or Tomato Plants or Plant Cells:

V-RNA particles produced as a result of the above procedures (as shown in 10a and 10c) can be engineered to contain the features necessary for replication and expression of the inserted foreign genetic information. Co-inoculation in step 11a, designated (11a) , of V-RNA particles, as shown in (10a) and (10c) , into plants or plant cells with complete TMV (which will supply the viral replicase) will result in replication of the V-RNA. Because the inserted genetic information (now in the form of RNA) lies within the coat protein gene of the V-RNA, the product P which the inserted genetic information codes for, will be produced in amounts comparable to that of the coat protein in complete TMV, and also the mode of production of m-RNA containing the inserted genetic information will essentially follow that for coat protein m-RNA production during replication of complete TMV. When the attached plasmid RNA is also encapsidated during vector particle formation, as in 10b above, if replication occurs, the attached plasmid RNA may be skipped over by the replicase enzyme during (-) and (+) strand synthesis as shown in step 11b, designated (11b) , and thus the plasmid RNA would be effectively lost to subsequent steps in virus synthesis, or such particles may not be replicated at all, in a manner analogous with that described with reference to step 3d and 3e, respectively, in figure 3. Under the former circumstances, the replicated V-RNA can be functional and direct the expression of the inserted foreign genetic information in a manner equivalent

-21-

to that of the V-RNA described above. The latter case will require a different method, such as that described with reference to step 10c, for recovery of the V-RNA.

Recovery of Replicated RNA Vector Particles from Infected Tobacco or Tomato Plant or Plant Cells:

The insertion of the foreign genetic information (gene) into the coat protein gene of the V-RNA will disrupt the continuity of the coat protein gene; therefore, the V-RNA will not synthesize functional coat protein. However, the co-infecting complete TMV will direct the production of function coat protein and both the V-RNA and TMV-RNA will be encapsidated, forming particles as shown in step 12, designated ⑫. The V-RNA particles can be isolated by standard procedures and can then be stored for future use in directing the replication and expression of the inserted foreign genetic information (gene) in plants or plant cells, such as tobacco or tomato.

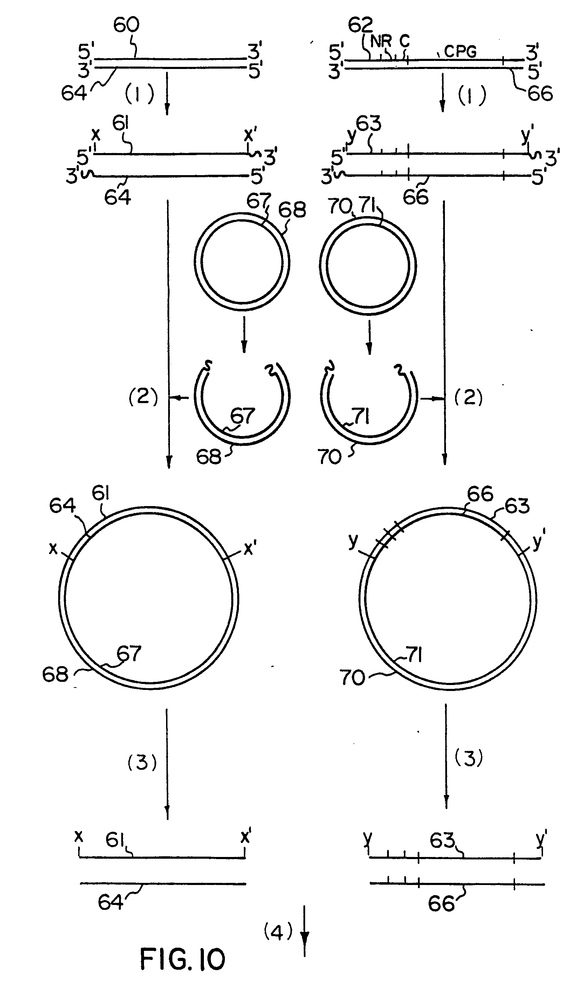

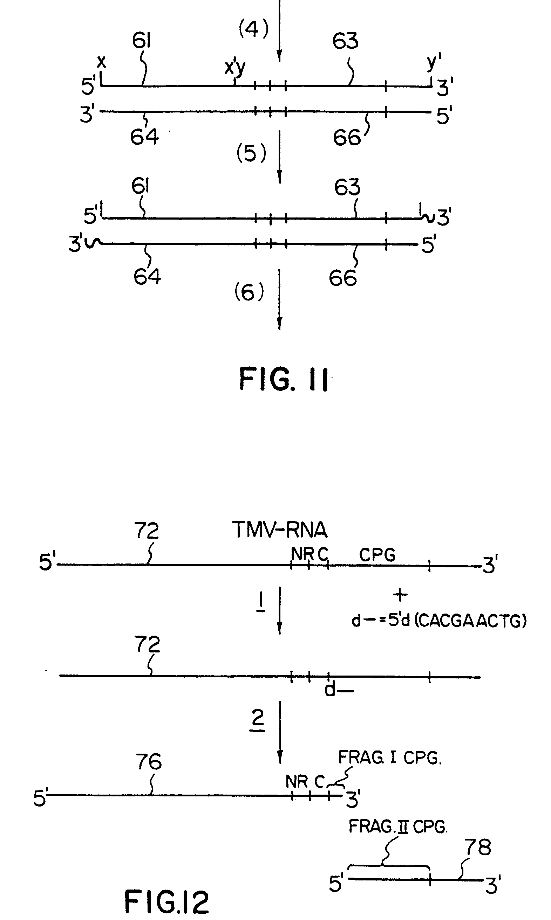

In figures 10 and 11, similar parts to those shown in figures 7 to 9 are designated in the same manner and the previous description is relied upon to describe them.

Example I

B. Generation and Isolation of Nucleotide Sequence Fragments of TMV-RNA in the form of Fragments I and II – Reverse Transcription of Fragments I and II – Insertion of the DNA Copies of Fragments I and II into Bacterial Plasmids – DNA-DNA Ligation of the DNA Copies of Fragments I and II – Insertion of the Ligated DNA Copies into Bacterial Plasmids – Insertion of a DNA Copy of Foreign Genetic Information (Gene) into the Vector Coat Protein Gene – Recovery of the RNA Vector Containing RNA Copy of the Inserted Foreign Information (Gene) – Replication of the RNA Vector and Expression of the Inserted Foreign Genetic Information (Gene) in Plants or Plant Cells, Such as Tobacco or Tomato – Recovery of Replicated RNA Vector Particles from Infected Plants or Plant Cells

Example IB will now be described with reference to figures 10 and 11.

-22-

Fragments I and II Generation and Isolation:

This is carried out in the same manner as has been described for Example IA for Fragment I, designated 60, and Fragment II, designated 62.

Reverse Transcription of Fragments I and II:

As shown in figure 10, the fragments appropriately primed at their 3' ends with a short complementary oligodeoxynucleotide are reverse-transcribed using avian myeloblastosis reverse transcriptase (BRL) resulting in the synthesis of single stranded DNA copies of Fragments I and II, designated 64 and 66 respectively. The ribo strand is then digested by alkaline treatment. The single stranded DNA copies 64 and 66 are then incubated with DNA polymerase I (BRL), in step 1 designated (1), to synthesis DNA double stranded copies of Fragments I and II, designated 61, 64, and dc-Frag. II-DNA, designated 63, 66 respectively, and the ends of these DNA copies are appropriately modified.

Insertion of the Double Stranded DNA Copies of Fragments I and II into Bacterial Plasmids

After modification, the double stranded DNA copies of Fragments I and II (61, 64, and 63, 66) are inserted in step 2, designated (2), into separate bacterial plasmids (e.g. pBR322) designated 67, 68, and 70, 71, respectively, by procedures analogous to those described in Example IA, steps (6a) and (6b) using a restriction endonuclease such as, for example Eco RI, to cleave the plasmids. The plasmids are then separately replicated, selected and amplified in the appropriate bacteria such as, for example E. coli. After purification of the plasmids, the double stranded DNA copies of Fragments I and II 61, 64 and 63, 66, respectively, are excised in step 3, designated (3), from the plasmids using a restriction endonuclease, such as, for example Eco RI.

It should be noted that x-x' represents the limits the double stranded DNA copy of Fragment I, 61, 64, and y-y' represents the limits of the double stranded DNA copy of Fragment II, 63, 66.

-23-

DNA-DNA Ligation of the Double Stranded DNA Copies of Fragments I and II

After purification, the double stranded DNA copies of Fragments I and II, 61, 64 and 63, 66, respectively, are then ligated together in step 4, designated (4), using DNA ligase. The product of the ligation step 4 is designated 61, 64, 63, 66 and is analogous to that in Example IA, step ④. The ends of the product 61, 64, 63, 66 are then appropriately modified in step 5, designated (5).

Insertion of the Ligated Double Stranded DNA Copies of Fragments I and II into Bacterial Plasmids

The modified product 61, 64, 63, 66, is then inserted into the appropriate plasmid (e.g. pBR322) by procedures analogous to Example IA, steps ⑥ⓐ and ⑥ⓑ. Procedures analogous to Example IA, steps ⑦ - ⑫ can now be followed for completion of Vector-RNA construction and for replication and expression of Vector-RNA in inoculated plants or plant cells such as tobacco or tomato.

It should be noted however, the cleavage of the plasmid prior to insertion is accomplished using a different restriction endonuclease to that previously used (e.g. Sal l). The modified product 61, 64, 63, 66, constructed as described will contain an Eco RI restriction site at the point of ligation if, as has been previously described, Eco RI is used in step (3). Because of the presence of the Eco RI restriction site the inserted DNA 61, 64, 63, 66, cannot be recovered intact using Eco RI to excise it from the plasmid as in Example IA, step ⑩. A Sal l site does not occur in the inserted DNA 61, 64, 63, 66, thus the inserted DNA 61, 64, 63, 66, can be excised from the plasmid using this restriction endonuclease. Conversely, if Sal l were used initially then, for example, Eco RI could be used at this stage.

Insertion of a DNA Copy of Foreign Genetic Information (Gene) into the Vector Coat Protein Gene

As in Example IA, steps ⑦ⓑ-⑨.

-24-

<u>Recovery of the RNA Vector Containing RNA Copy of the Foreign Genetic</u>
<u>Information (Gene)</u>

As in Example IA, steps ⑨ , ⑩ⓐ , ⑩ⓑ and ⑩ⓒ .

<u>Replication of the RNA Vector and Expression of the Foreign Genetic</u>
<u>Inforamtion (Gene) in Plants or Plant Cells such as Tobacco or Tomato</u>

As in Example IA, steps ⑪ⓐ and ⑪ⓑ .

<u>Recovery of Replicated RNA Vector Particles from Infected Plants or Plant</u>
<u>Cells such as Tobacco and Tomato</u>

As in Example IA, step ⑫ .

In figures 12 to 15, similar parts to those shown in figures 1 to 9 are designated in the same manner and the previous description is relied upon to describe them.

<div align="center">Example II</div>

<u>Generation and Isolation of Nucleotide Sequence Fragments I and II from</u>
<u>TMV-RNA - Extension of the 3' end of Fragment I - Reverse Transcription</u>
<u>of Fragment II in Preparation for Ligation - Reverse Transcription of</u>
<u>Foreign Genetic Information (m-RNA) - Insertion and Ligation of the m-RNA</u>
<u>into the Vector-RNA Coat Protein Gene - Replication of the RNA Vector (V</u>
<u>RNA) and Expression of the Inserted Gene in Plants or Plant Cells such as</u>
<u>Tobacco or Tomato - Recovery of Replicated RNA Vector Particles from</u>
<u>Infected Plants or Plant Cells</u>

<u>Generation and Isolation of Fragments I and II</u>

As shown in figure 12, the TMV-RNA 72 is mixed with an oligodeoxynucleotide - 5'd (CACGAACTG), shown as "d-". The mixture is incubated in step 1 at 55°C for 15-30 min. and then allowed to cool slowly. Under these conditions, the oligodeoxynucleotide will anneal to its complementary sequence in the TMV-RNA. In this example, the unique 5'r (CAGUUCGUG) is located in the coat protein gene (CPG). This nucleotide sequence which codes for amino acids 9-11 in TMV coat protein is as follows:

<div align="center">-25-</div>

## Fragment II

5' AUUGUUUAUAGAAAUAAUAUAAAAUUAGGUUUGAGAGAGAⱭGAUUACAAGCGUGAGAGACGGAGGG

CCCAUGGAACUUACAGAAGAAGUUGUUGAUGAGUUCAUGGⱭAGAUGUCCCUAUGUCAAUCAGACUU

─────────Nucleation Region─────────

GCAAAGUUUCGAUCUCGAACCGGAAAAAAGAGUGAUGUCCGUAAAGGGAAAAUUAGUAGUAGUGAU

CGGUCAGUGCCGAⱭCAAGAACUAUAGAAAUGUUAAGGAUUⱭUGGAGGAAUGAGUUUUAAAAAGAAU

·············· Control Region··························

AAUUUAAUCGAUGAUGAUUCGGAGGCUACUGUCGCCGAAUCGGAUUCGUUUUAAAUAUGUCUUACA

GUAUCACUACUCCAUCUCAGUUCGUGUUCUUGUCAUCAGCGUGGGCCGACCCAAUAGAGUUAAUUA

AUUUAUGUACUAAUGCCUUAGGAAAUCAGUUUCAAACACAⱭCAAGCUCGAACUGUCGUUCAAAGAC

AAUUCAGUGAGGUGUGGAAACCUUCACCACAAGUAACUGUUAGGUUCCCUGACAGUGACUUUAAGG

──────────Coat Protein Gene──────────

UGUACAGGUACAAUGCGGUGUUAGACCCGCUAGUCACAGCⱭUUACUAGGUGCAUUUGACACUA

GAAAUAGAAUAAUAGAAGUUGAAAAUCAGGCGAACCCCACGACUGCCGAAACGUUAGAUGCUACUC

GUAGAGUAGACGACGCGACGGUGGCCAUAAGGAGCGCGAUAAAUAAUUUAAUAGUAGAAUUGAUCA

GAGGAACCGGAUCUUAUAAUCGGAGCUCUUUCGAGAGCUCⱭUCUGGUUUGGUUUGGACUUCCGGUC

CUGCAACUUGAGGUAGUGCAACUUGAGGUAGUCAAGAUGCⱭꓕAAUAAAUAACGGAUUGUGUCCGUA

AUCACACGUGGUGCGUACGAUAACGCAUAGUGUUUUUUCCCUCCACUUAAAUCGAAGGGUUGUGUCU

UGGAUCGCGCGGGUCAAAUGUAUAUGGUUCAUAUACAUCCGCAGGCACGUAAUAAAGCGAGGGGUU

CGAAUCCCCCCGUUACCCCCGGUAGGGGCCCA-OH    3'

Note: The underlined nucleotide sequence is the nucleotide sequence cleaved by ribonuclease H.

After annealing, ribonuclease H (P-L Biochemicals) is added in step 2 and incubation continued for 30 min. This enzyme specifically degrades RNA in RNA-DNA hybrid structures. TMV-RNA is thus cleaved into two fragments, namely Fragment I (designated 76) and Fragment II (designated 78), the cleavage point being a specific site in the CPG as indicated above. The larger, Fragment I 76, is composed of approximately 5742 nucleotides, originating at the capped 5' end of the viral RNA and extending into the coat protein gene, designated Frag.I CPG. The viral replicase gene is likely included in Fragment I. The smaller, Fragment II 78, is composed of approximately 663 nucleotides, originating at the 5' end of the cleavage site in the coat protein gene, designated Frag. II CPG, and extending to the 3' end of the viral RNA.

This partial nucleotide sequence of the Fragment I 76, which includes Fragment I of Example IA, is as follows:

```
        |←——Frag.I (Ex.IA)——→|
        |              70|                      5400
m⁷GpppG|.................AUG|.....N(N₅₃₃₄)N......AUUGUUUAUAGAAAUAAU

                                   |←———————————————————————————
AUAAAAUUAGGUUUGAGAGAGAAGAUUACAAGCGUGAGAGACGGAGGGCCCAUGGAACUUACAGAAGAA

———————————————————————————————————————Nucleation Region—————————
                5500
GUUGUUGAUGAGUUCAUGGAAGAUGUCCCUAUGUCAAUCAGACUUGCAAAGUUUCGAUCUCGAACCGGA

——————————————————————————————————————————————————————————————————
                                           5600
AAAAAGAGUGAUGUCCGUAAAGGGAAAAUUAGUAGUAGUGAUCGGUCAGUGCCGAACAAGAACUAUAGA

————————→|←·············································Control Region···············
AAUGUUAAGGAUUUUUGGAGGAAUGAGUUUUUAAAAAGAAUAAAUUUAAUCGAUGAUGAUUCGGAGGCUACU

··························→|←— Coat Protein Gene ——
                5700
GUCGCCGAAUCGGAUUCGUUUUAAAUAUGUCUUACAGUAUCACUACUCCAUCU|CAGUUCGUG|  3'
```

where [    ] is the site of cleavage by ribonuclease H.

-27-

The nucleotide sequence of Fragment II, designated 78 is as follows:

UUCUUGUCAUCAGCGUGGGCCGACCCAAUAGAGUUAAUUA

AUUUAUGUACUAAUGCCUUAGGAAAUCAGUUUCAAACACAACAAGCUCGAACUGUCGUUCAAAGAC

AAUUCAGUGACCUGUGGAAACCUUCACCACAAGUAACUGUUAGGUUCCCUGACAGUGACUUUAAGG

————————————Coat Protein Gene——————————————

UGUACAGGUACAAUGCGGUGUUAGACCCGCUAGUCACAGCAUUACUAGGUGCAUUUGACACUA

GAAAUAGAAUAAUAGAAGUUGAAAAUCAGGCGAACCCCACGACUGCCGAAACGUUAGAUGCUACUC

GUAGAGUAGACGACGCGACGGUGGCCAUAAGGAGCGCGAUAAAUAAUUUAAUAGUAGAAUUGAUCA

GAGGAACCGGAUCUUAUAAUCGGAGCUCUUUCGAGAGCUCUUCUGGUUUGGUUUGGACUUCCGGUC

CUGCAACUUGAGGUAGUGCAACUUGAGGUAGUCAAGAUGCAUAAUAAAUAACGGAUUGUGUCCGUA

AUCACACGUGGUGCGUACGAUAACGCAUAGUGUUUUUCCUCCACUUAAAUCGAAGGGUUGUGUCU

UGGAUCGCGCGGGUCAAAUGUAUAUGGUUCAUAUACAUCCGCAGGCACGUAAUAAAGCGAGGGGUU

CGAAUCCCCCCGUUACCCCCGGUAGGGGCCCA-OH    3'

<u>Extension of the 3' End of Fragment I</u>

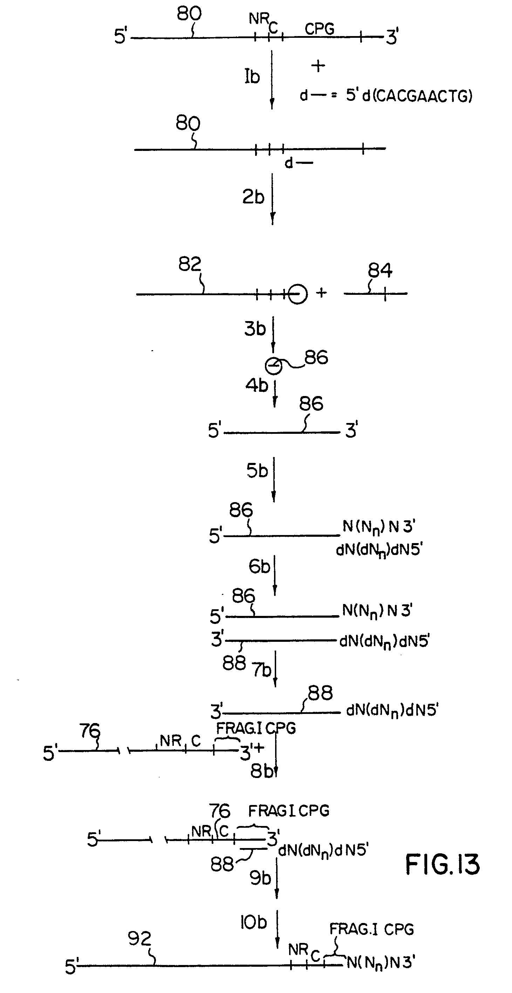

As shown in figure 13, a Fragment II RNA, designated 80, produced as described with reference to Fragment II RNA in Example IA, is mixed and allowed to anneal with the oligodeoxynucleotide described in Example II, step 1b. Ribonuclease H is then added in step 2b and after incubation the two RNA segments produced, designated 82 and 84, are isolated and purified. The segment 82, corresponding to the 3' end region of Fragment I in figure 12 is then digested in step 3b with ribonuclease $T_1$ (Sigma Chemical Co.). The oligoribonucleotide 5' (UAUCACU-ACUCCAUCU) 3', designated 86, is then isolated in step 4b. This oligonucleotide has the same nucleotide sequence as that of the 3' end of Fragment I, designated 76. This oligonucleotide is then extended in step 5b in the 3' direction using polynucleotide phosphorylase (P-L Biochemicals) and the appropriate ribonucleotide diphosphate (rNDP). The extended oligoribonucleotide is then reverse-transcribed in step 6b using reverse transcriptase and an appropriate primer such as, for example, oligo $(dT)_{10\ 12}$ (P-L Biochemical). The ribo strand 86 is then digested by alkaline treatment in step 7b and the deoxy strand (c-DNA strand) 88 is annealed in step 8b to Fragment I 76 (figure 12) from step 2 described above. Using the unannealed 5' end of the c-DNA strand 88 as a template, Fragment I is extended in step 9b in the 3' end direction using DNA polymerase I (BRL) and the appropriate ribonucleotide triphosphate (rNTP). The c-DNA strand is then digested in step 10b with DNAase leaving Fragment I, designated 92, which has been extended in the 3' end direction.

Reverse Transcription of Fragment II in Preparation for Ligation

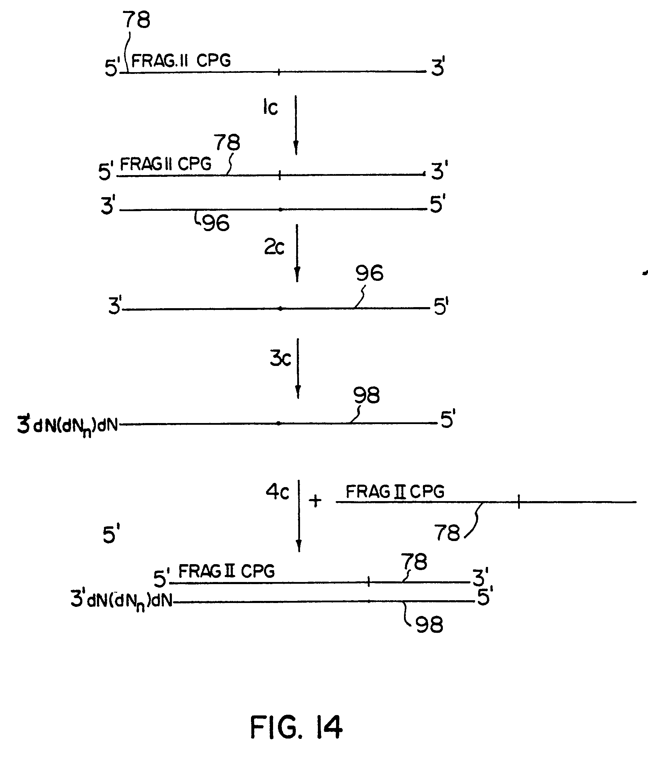

As shown in figure 14, Fragment II, designated 78, generated in step 2 of figure 12, is primed in step 1c with an oligodeoxynucleotide complementary to its 3' end and reverse-transcribed using reverse transcriptase. The ribo strand 78 is then digested in step 2c by alkaline treatment and the single stranded DNA copy of Fragment II, designated 96, isolated and extended in step 3c in the 3' end direction using terminal deoxynucleotidyl transferase (BRL) and the appropriate deoxynucleotide triphosphate (dNTP). The extended DNA copy 98 is then annealed in step 4c with Fragment II, designated 78, which is generated in step 2, figure 12, thus forming a Fragment II RNA/DNA hybrid 78, 98.

-29-

Reverse Transcription of m-RNA in Preparation for Insertion in the Coat
Protein Gene

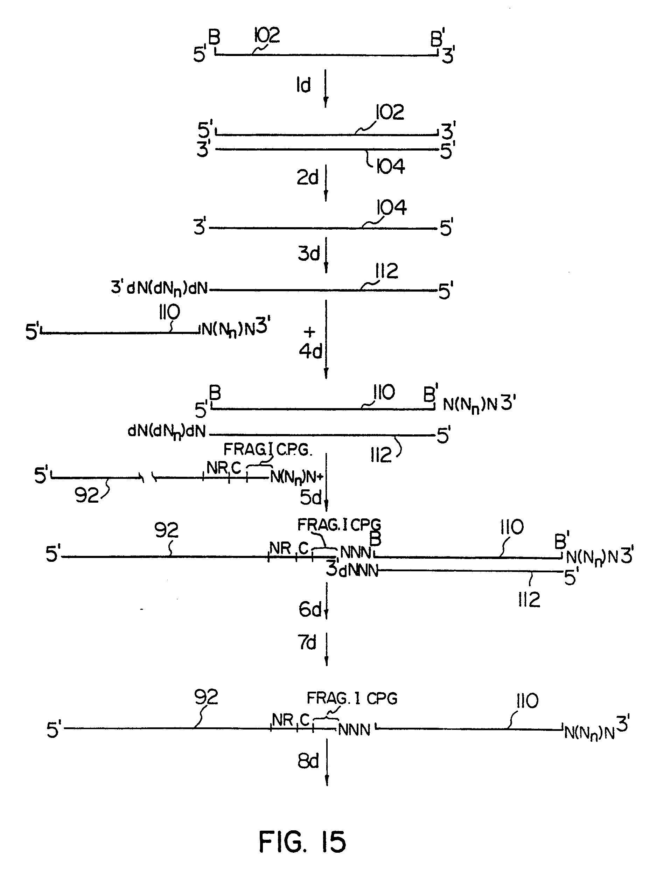

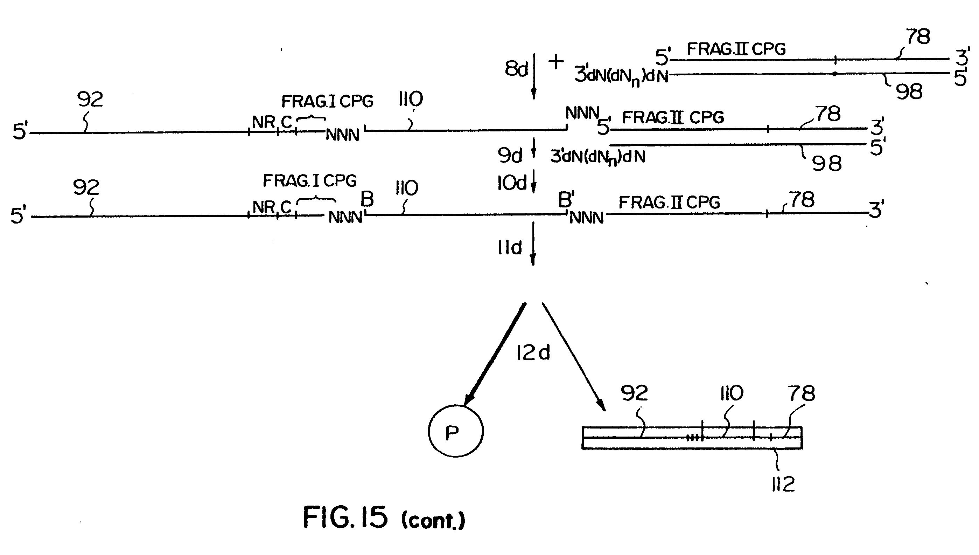

As shown in figure 15, purified m-RNA, designated 102, is
reverse-transcribed in step 1d using reverse transcriptase. The ribo
strand 102 is then digested in step 2d by alkaline treatment and the DNA
copy of the m-RNA 104 is then extended in step 3d in the 3' end direction
using terminal deoxynucleotidyl transferase and the appropriate deoxynucleotide triphosphate (dNTP) to form strand strand 112. The extended DNA
strand 112 is then annealed in step 4d with m-RNA, designated 110, which
has been previously extended at its 3' end using the same procedure as in
the extension of the oligonucleotide 86 as described in step 5b with
reference to figure 13, thus forming an m-RNA/DNA hybrid 110, 112.

Insertion and Ligation of the m-RNA into the Vector-RNA Coat Protein
Gene

The extended Fragment I, designated 92, and the m-RNA/DNA
hybrid 110, 112 are mixed together in step 5d, figure 15, and incubated
with DNA polymerase I and the appropriate ribonucleotide triphosphate
(rNTP) in step 6d. The extended region at the 3' end of Fragment I,
designated 92, will anneal with the extended region at the 3' end of the
DNA 112 of the hybrid, thus bringing the 3' end of the Fragment I 92 and
the 5' end of the m-RNA 110 into alignment; the DNA polymerase will fill
in the gap between the aligned RNA molecules. DNA ligase is then used in
step 7d to covalently ligate the 3' end of Fragment I, designated 92, to
the 5' end of the m-RNA 110. The DNA 112 of the hybrid is then digested
with DNAase. The Fragment I-m-RNA 92, 110 is then mixed in step 8d with
the Fragment II RNA/DNA hybrid 78, 98, in figure 14. The extended 3' end
of the Fragment I-m-RNA 92, 110 and the 5' end of Fragment II 78, are
brought into alignment by the extended 3' end of the DNA 98 of the
hybrid. DNA polymerase and DNA ligase are then used in step 9d to
covalently ligate the 3' end of the Fragment I-m-RNA molecule, designated

-30-

92, 110, to the 5' end of the Fragment II 78. DNAase is then used to digest the DNA 98 of the Fragment II RNA/DNA hybrid 78, 98. The Fragment I-m-RNA-Fragment II molecule (V-RNA) is then encapsidated in step 11d with TMV coat protein, designated 112, and inoculated into plants or plant cells in step 12d for replication and expression resulting in the production of at least one gene derived product, designated P, and at least a portion of a vector 92, 110, 78, for, for example, the purposes which will be described later.

Replication of the RNA Vector and Expression of the Inserted Gene in Plants or Plant Cells such as Tobacco or Tomato

V-RNA particles produced according to the present invention, as previously described, can contain the features necessary for replication and expression of the inserted foreign genetic information. This V-RNA can also contain the gene for synthesis of the viral replicase and therefore be able to replicate in, for example, tobacco without the need of co-infection with complete TMV. (The V-RNA produced in Example I would not contain the replicase gene and thus would require co-infection with TMV for replication.) Expression of the inserted m-RNA (foreign gene information) can follow the same course as set out in Example I with product yield equivalent to the yield coat protein synthesized during infection of, for example, tobacco with TMV. Note: The mode of Fragment I and II production as set forth in this Example II enables more flexibility in that the fragments of any desired length from any location in TMV-RNA can be generated, see, for example, the following fragment which may be produced in this manner:

|——Frag.I (Ex.IA)————————|
```
                                          70              90
m⁷GpppG..................AUGGCAUACACACAGACAGCUACCACAUCAGCUUUGCUG
```

```
    110              130              150    GUCUUUA    170
GACACUGUCCGAGGAAACAACUCCUUGGUCAAUGAUCAAGCAAGCGUC       CGACACAGCGGU
                                               UUUACGU
```

```
        190              210              230
UGAGAAGUGCUCGUUUAACGACCGCAGGCCCAAAGUGAACUUUUCAAAAGUAAUAAGCGAG    3'
```

Also, this approach allows for the insertion of foreign genetic information into virtually any location within the vector RNA. In Example I, the insertion point is fixed in that only sequences which contain restriction endonuclease sites can be used as insertion points.

Recovery of Replicated RNA Vector Particles from Infected Plants or Plant Cells such as Tobacco or Tomato

As in Example I, insertion of the foreign genetic information into the coat protein gene will prevent the production of functional coat protein, because V-RNA produced in Example II will not require co-infection with complete TMV for viral replicase production, there will not be any functional coat protein produced; therefore, V-RNA will not be encapsidated into particle form. Recovery of V-RNA will require isolation of total RNA followed by incubation with TMV coat protein. The V-RNA will be specifically encapsidated (by virtue of containing the NR sequences) and the V-RNA particles formed can be isolated by standard procedures. A different method is to co-infect with complete TMV in order to cause formation of vector particles within the plants or plant cells. Particles produced in either manner can be stored for future use in directing the replication and expression of foreign genetic information in plants or plant cells.

-32-

## TABLE OF EXAMPLES OF RNA PLANT VIRUSES

1.  Tobacco Rattle Virus

2.  Tobacco Mosaic Virus

3.  Potato Virus X

4.  Carnation Latent Virus

5.  Potato Virus Y

6.  Alfalfa Mosaic Virus

7.  Pea Enation Mosaic Virus

8.  Cucumber Mosaic Virus

9.  Turnip Yellow Mosaic Virus

10. Cowpea Mosaic Virus

11. Tobacco Ringspot Virus

12. Tobacco Necrosis Virus

13. Brome Mosaic Virus

14. Tomato Bushy Stunt Virus

15. Tomato Spotted Wilt Virus

## TABLE OF EXAMPLES OF TMV STRAINS

| Name | Host |
|---|---|
| Common mosaic [vulgar (U 1)] | tobacco |
| Aucuba mosaic (YA) | tomato |
| Cucumber mosaic 3 (CV 3) | cucumber |
| Cucumber mosaic 4 (CV 4) | cucumber |
| Dahlemense | tomato |
| Holmes' rib grass (HR) | rib grass |
| Southern sunnhemp mosaic (SSM) | sunn hemp |
| Mild Mosaic (U 2) | tobacco |
| Odontoglossum ringshot (ORSV) | orchid |
| (U 5) | tobacco |
| (O M) | tobacco |
| Cowpea (CPV) | cowpea |
| Sammons' Opuntia (SOV) | cactus |
| Yellow tomato atypical mosaic (Y-TAMV) | tomato |
| Green tomato atypical mosaic (G-TAMV) | tomato |
| (CV 60) | tomato |
| (CV 61) | tomato |
| American collection no. 9 (AC-9) | tomato |
| Hall-Davis (HD) | tomato |
| San Joaquin (S J) | tomato |
| Ventura (VEN) | tomato |
| Australia II (Aus-II) | tomato |
| Dutch I (Dut-I) | tomato |
| (K-1) | tomato |
| (PTA) | tomato |
| Philippine (PTV) | tomato |
| South African (SAF) | tomato |
| Cucumber green mottle mosaic (CGMMV) | cucumber |
| (01 through 07) | orchids |
| (B-TMV 1 and 2) | pear tree |
| Yellow leaf G.P. (YLGP) | tomato |

The following desirable features of the present invention should be noted:

1. The RNA Vector, constructed as previously described in accordance with the present invention, will direct the replication and expression of the inserted foreign genetic information. Because such insertions can be in the coat protein gene, it follows that the inserted gene product will be synthesized at yields comparable to those of coat protein synthesized by complete TMV during the infection process. Such yields are in the range of 1-10 g coat protein/kg (fresh weight) leaf tissue. While direct comparison of these yields to those obtained in known DNA plasmid/bacteria systems cannot be made, the following will give an approximate comparison of expected yields by the two systems:

RNA Vector in tobacco plants (yield of product based on yield of TMV coat protein obtained in tobacco):

$10^7$ viral particles/leaf cell x $10^3$ coat protein molecules/viral particle = $10^{10}$ coat protein molecules/cell

$10^{10}$ coat protein molecules/cell x $10^6$ cells/g (fresh weight) leaf tissue = $10^{16}$ coat protein molecules/g leaf tissue

$10^{16}$ coat protein molecules/g leaf tissue x $10^3$ g/kg = $10^{19}$ coat protein molecules/kg leaf tissue

(1 g coat protein/kg leaf tissue)

DNA plasmid in bacteria [yield based on $10^5$ interferon or $10^5$ insulin molecules/cell (See: N. Wade, Science 208, 1441, 1980; and D. Goeddel et al, Proc. Nat. Acad. Science 76, 106-110, 1979)].

$10^8$ bacteria/ml x $10^5$ interferon (insulin) molecules/cell = $10^{13}$ molecules/ml

$10^{13}$ molecules/ml x $10^3$ ml/litre = $10^{16}$ molecules/litre

$10^{16}$ molecules/l x $10^3$ litres = $10^{19}$ molecules/1000 litres

-35-

2.    Many of the products which have commercial application are the products of eucaryotic genes. The expression of these genes in the DNA plasmid/bacteria system (procaryotic) has already presented problems. It has also been reported that the products of eucaryotic genes expressed in bacteria are degraded by bacterial proteases. The RNA Vector/plant system made possible by the present invention is a eucaryotic system and it therefore follows that it is compatible with the eucaryotic genes to be inserted, and their products.

3.    Some of the products of eucaryotic genes produced in the DNA plasmid/bacteria system are incomplete. For example, interferon and antibody molecules synthesized in the bacteria system are not glycosylated (specific sugar molecules added to the protein). It is believed that such addition occurs in the endoplasmic reticulum system in eucaryotic cells. bacteria lack such a system but it is present in plant cells.

4.    The RNA vector system made possibly by the present invention, functions at or near room temperature (25°C); the bacterial system functions near 37°C. Labile proteins, etc., are likely to be more stable at the temperature used in the RNA vector/plant system than in the bacterial system.

5.    Often there are toxic or pyrogenic compounds (polysaccharides) produced by bacteria. Such compounds must be completely removed from the product if it is to be used in humans. There is much less likelihood that similar compounds will be present in the RNA vector/plant system made possible by the present invention.

6.    The RNA vector according to the present invention is not known to be capable of replication in animal or bacterial cells; therefore, it presents a significantly lower biological hazard potential than the DNA plasmid system. The biological containment factor can also be significantly increased by deletion of the nucleation region necessary for encapsidation. The vector-RNA, according to the present invention, cannot survive outside the plant cell if not encapsidated by viral protein;

-36-

therefore, the likelihood of inadvertant release is further minimized.

The following uses of the present invention are given by way of example:

1.  The RNA vector/plant system made possible by the present invention will have the same scope of potential as the DNA plasmid/bacteria system, e.g. the synthesis of proteins, enzymes, antibodies, hormones (insulin, somatostatin, growth hormone), nucleotides, polynucleotides, antigenic proteins, anti-viral compounds (interferon), primary and secondary metabolites.

2.  The RNA vector, according to the present invention, may have application in altering the metabolism of plants or plant cells by directing the synthesis of enzymes necessary for the synthesis and accumulation of primary and secondary products in plants or plant cells.

3.  The RNA vector, according to the present invention, may have application in altering the metabolism of plants or plant cells, leading to advantageous changes, such as, growth habit, yield potential, energy utilization.

4.  The RNA vector, according to the present invention, may have direct application in improving disease resistance of plants and improving resistance to environmental stress conditions (cold hardiness, increased salt tolerance, etc.).

CLAIMS:

1.    A method of constructing an RNA plant virus vector or portion thereof from TMV and other RNA plant viruses, for the insertion of foreign genetic information therein, and the transfer into and replication and expression of the vector or portion thereof, with inserted foreign genetic information, by self-replication, or replication upon co-inoculation with helper virus, in plants or plant cells, which comprises, combining a nucleotide sequence originating from the 5' end of the plus (+) strand of the viral RNA and a nucleotide sequence originating from the 3' end of the plus (+) strand of the viral RNA, such nucleotide sequences being hereinafter referred to as fragments and being selected from the group consisting of oligonucleotides and polynucleotides,

(a) the fragment originating from the 5' end being hereinafter referred to as Fragment I, and extending in the 3' end direction and comprising those nucleotide sequences complementary to the recognition and binding sites for the viral polymerase in the minus (-) strand of viral RNA, and

(b) the fragment originating from the 3' end being hereinafter referred to as Fragment II, and extending in the 5' end direction and comprising those nucleotide sequences for the recognition and binding of the viral polymerase in the plus (+) strand of viral RNA, and wherein

(c) at least one of the Fragments I and II contains at least a portion of the viral coat protein gene, and

(d) the Fragments I and II alone or in combination contain a nucleotide sequence in the form of a control region or portion thereof, which will control the expression of the coat protein gene.

2.    The method as claimed in claim 1 wherein the fragments are generated such that the Fragments I and II, alone or in combination, contain a nucleotide sequence or portion thereof forming a nucleation region or portion thereof, necessary for the encapsidation of the RNA by viral coat protein.

3.    The method as claimed in claim 1 which further includes the insertion or attachment of foreign genetic information, ultimately in the form of RNA, to the said RNA vector or portion thereof.

-38-

CLAIMS (cont.)

4.       The method as claimed in claim 3 wherein the genetic information is inserted into or attached to the coat protein gene or portion thereof.

5.       The method as claimed in claim 3 wherein the foreign genetic information is inserted into or attached to the control region or portion thereof.

6.       The method as claimed in claim 2 which further includes the insertion or attachment of foreign genetic information, ultimately in the form of RNA, to the nucleation region or portion thereof of the said RNA vector or portion thereof.

7.       The method as claimed in claim 1 or 2 wherein the RNA plant virus vector or portion thereof is generated for the purpose of reproduction by inoculation into plants or plant cells.

8.       The method as claimed in claim 3, 4, 5 or 6 wherein the said RNA plant virus vector or portion thereof, with added foreign genetic information, is generated for the purpose of reproduction by inoculation into plants or plant cells.

9.       The method as claimed in claim 3, 4, 5 or 6 wherein the said RNA plant virus vector or portion thereof, with added foreign genetic information, is generated for the purpose of directing the synthesis of a product, selected from the group consisting of proteins, oligonucleotides, polynucleotides, peptides, enzymes, antibodies, antigenic substances, anti-viral compounds, anti-cancer compounds and primary and secondary metabolites upon inoculation into plants or plant cells.

10.      The method as claimed in claim 3, 4, 5 or 6 wherein the said RNA plant virus vector or portion thereof, with added foreign genetic information, is generated for the purpose of altering the metabolic or catabolic capability of plants or plant cells upon inoculation into plants or plant cells.

-39-

0067553

CLAIMS (cont.)

11.     The method as claimed in claim 3, 4, 5 or 6 wherein the said RNA plant virus vector or portion thereof, with added foreign genetic information, is generated for the purpose of altering at least one of the group consisting of growth habit, yield potential, disease resistance, resistance to environmental stress, and energy utilization of the plants or plant cells upon inoculation into plants or plant cells.

12.     An RNA plant virus vector or portion thereof derived from TMV and other RNA plant viruses, for the insertion of foreign genetic information therein, and the transfer into and replication and expression of the vector of portion thereof, with inserted foreign genetic information by self-replication, or replication upon co-inoculation with helper virus, in plants or plant cells, the RNA plant virus vector or portion thereof comprising a nucleotide sequence originating from the 5' end of the plus (+) strand of the viral RNA and a nucleotide sequence originating from the 3' end of the plus (+) strand of the viral RNA, the nucleotide sequences being hereinafter referred to as fragments and being combined and selected from the group consisting of oligonucleotides and polynucleotides,

(a)  the fragment originating from the 5' end being hereinafter referred to as Fragment I and extending in the 3' end direction and comprising those nucleotide sequences complementary to the recognition and binding sites for the viral polymerase in the minus (-) strand of viral RNA, and

(b)  the fragment originating from the 3' end being hereinafter referred to as Fragment II, and extending in the 5' end direction and comprising those nucleotide sequences for the recognition and binding of the viral polymerase in the plus (+) strand of viral RNA, and wherein

(c)  at least one of the Fragments I and II contains at least a portion of the viral coat protein gene, and

(d)  the Fragments I and II alone or in combination contain a nucleotide sequence in the form of a control region or portion thereof, which will control the expression of the coat protein gene.

-40-

CLAIMS (cont.)

13.     The RNA plant virus vector or portion thereof as claimed in claim 12 further comprising a nucleotide sequence or portion thereof forming a nucleation region or portion thereof, necessary for the encapsidation of the RNA by viral coat protein.

14.     The RNA plant virus vector or portion thereof as claimed in claim 12, which further includes an insertion or attachment of foreign genetic information, ultimately in the form of RNA.

15.     The RNA plant virus vector or portion thereof as claimed in claim 14 wherein the foreign genetic information has been inserted into or attached to the coat protein gene or portion thereof.

16.     The RNA plant virus vector or portion thereof as claimed in claim 14 wherein the foreign genetic information has been inserted into or attached to the control region or portion thereof.

17.     The RNA plant virus vector or portion thereof as claimed in claim 13 which further includes an insertion or attachment of foreign genetic information, ultimately in the form of RNA, to the nucleation region or portion thereof.

18.     The RNA plant virus vector or portion thereof as claimed in claim 14, 15, 16 or 17 wherein the said RNA plant virus vector or portion thereof with the added foreign genetic information is for the purpose of directing the synthesis of a product, selected from the group consisting of oligonucleotides, polynucleotides, proteins, peptides, enzymes, antibodies, antigenic substances, anti-viral compounds, anti-cancer compounds and primary and secondary metabolites upon inoculation into plants or plant cells.

19.     The RNA plant virus vector or portion thereof as claimed in claim 14, 15, 16 or 17 wherein the said RNA plant virus vector or portion thereof, with the added foreign genetic information, is for the purpose of altering the metabolic or catabolic capability of plants or plant cells upon inoculation into plants or plant cells.

-41-

CLAIMS (cont.)

20.    The RNA plant virus vector or portion thereof as claimed in claim 14, 15, 16 or 17 wherein the said RNA plant virus vector or portion thereof, with the added foreign genetic information, is for the purpose of altering at least one of the group consisting of growth habit, yield potential, disease resistance, resistance to environmental stress and energy ultilization of the plants or plant cells upon inoculation into plants or plant cells.

21.    A method of producing a gene-derived product comprising, inoculating the plant or plant cell with an RNA plant virus vector or portion thereof derived from TMV and other RNA plant viruses, the vector or portion thereof having foreign genetic information inserted therein, and being inoculated into the plant or plant cells for the replication and expression of the vector or portion thereof, with inserted genetic information, leading to the production and accumulation of the said gene-derived product, by self-replication, or replication upon co-inoculation with helper virus, in plants or plant cells, the RNA plant virus vector or portion thereof comprising a nucleotide sequence originating from the 5' end of the plus (+) strand of the viral RNA and a nucleotide sequence originating from the 3' end of the plus (+) strand of the viral RNA, the nucleotide sequences being hereinafter referred to as fragments and being combined and selected from the group consisting of oligonucleotides and polynucleotides,

(a)  the fragment originating from the 5' end being hereinafter referred to as Fragment I and extending in the 3' end direction and comprising those nucleotide sequences complementary to the recognition and binding sites for the viral polymerase in the minus (-) strand of viral RNA, and

(b)  the fragment originating from the 3' end being hereinafter referred to as Fragment II, and extending in the 5' end direction and comprising those nucleotide sequences for the recognition and binding of the viral polymerase in the plus (+) strand of viral RNA, and wherein

-42-

CLAIMS (cont.)

21.(cont.)

(c)  at least one of the Fragments I and II contains at least a portion of the viral coat protein gene, and

(d)  the Fragments I and II, alone or in combination, contain a nucleotide sequence in the form of a control region or portion thereof, which will control the expression of the coat protein gene.

FIG. 1

**FIG. 2**

(a) 5' ——————4—— TMV-RNA ———————OH 3'
     6 ⊘ 10

(b) 5' ——————4———————————OH 3'
     ⊘ 6 10

(c) 5" ——————4———————— 10 AAA(An)A 3'

(d) 5' ——————4———————— 10 AAA(An)A 3'
     ⊘ 6 10    ⊘ 6

(e) 5' ——————4———— X 10 AAA(An)A 3'

FIG. 3

13    4    NR C  CPG   10
5' ——————————————————— 3'
FRAG I (EX.I)    FRAG.II (EX.I)
FRAG. I (EX.2)   FRAG II (EX 2)

FIG. 4

(a) 5' ——————————— NR C  CPG ———— 3'

(b) 5' ——————————— NR C ————— 3'
                        mRNA
                        CPG

FIG. 5

(a) 5' ——————————— NR C  CPG ———— 3'

(b) 5' ——————————— C  CPG ———— 3'
                   NR

FIG. 6

3/12

0067553

0067553

FIG. 7

FIG. 8

FIG.9

FIG. 10

FIG. 11

FIG. 12

FIG.13

78

5' FRAG.II CPG ————————————————|————————————— 3'

1c ↓

78
5' FRAG II CPG ————|———————————— 3'
3' ————————————————|———————————— 5'
        96

2c ↓

96
3' ————————————————|——————————— 5'

3c ↓

98
3' dN(dNₙ)dN ————————|——————————— 5'

4c ↓ +    FRAG II CPG
                ——————————————|—————————————
5'                      78

5' FRAG II CPG ————————————|———— 3'
3' dN(dNₙ)dN ——————————————————— 5'
                        98

FIG. 14

FIG. 15

FIG. 15 (cont.)